# EUROPEAN PATENT APPLICATION

(11) **EP 4 786 592 A1**
(43) Date of publication of application: **05.08.2026**
(21) Application number: 24873054.1
(22) Date of filing: 27.09.2024
(51) Int. Cl.: C12N 15/11, C12N 15/113, C12N 15/67, C07K 14/705, A61K 48/00, A61P 35/00, A61K 38/00

(54) **NUCLEIC ACID MOLECULE COMPRISING MODIFIED POLYADENYLATION SEQUENCE AND OX40L PROTEIN-CODING SEQUENCE**

(30) Priority: 27.09.2023 KR 20230130731
(71) Applicant: Hanmi Pharm. Co., Ltd., Hwaseong-si, Gyeonggi-do 18536 (KR)
(72) Inventor: KIM, Jeong Woo, Hwaseong-si, Gyeonggi-do 18469 (KR); SHIN, Seung Hyun, Hwaseong-si, Gyeonggi-do 18469 (KR); LEE, Jong Soo, Hwaseong-si, Gyeonggi-do 18469 (KR); LIM, Chang Gyu, Hwaseong-si, Gyeonggi-do 18469 (KR); JANG, Doo Seo, Hwaseong-si, Gyeonggi-do 18469 (KR); HAN, Young Jin, Hwaseong-si, Gyeonggi-do 18469 (KR); HEO, Yong Ho, Hwaseong-si, Gyeonggi-do 18469 (KR)
(74) Representative: Santarelli
(86) International application number: PCT/KR2024/014757
(87) International publication number: WO 2025/071347

(57) **Abstract**

Provided are an mRNA molecule including a coding sequence for a polypeptide containing the extracellular domain of OX40L and a modified polyadenyl sequence, a composition for delivering the mRNA or a product expressed thereby to a subject, a method of delivering the mRNA or a product expressed thereby to a host cell, and a nucleic acid molecule encoding the mRNA molecule.

## Description

### Technical Field

The present disclosure relates to a nucleic acid molecule including a modified polyadenyl sequence and an OX40L protein-coding sequence, a nucleic acid molecule encoding the same, and uses thereof.

### Background Art

RNA, such as *in vitro* transcribed RNA (IVT-RNA), is being proposed as an alternative to the therapeutic use of DNA. However, the injection of RNA for clinical application may be significantly limited by the short half-life of RNA.

Additionally, IVT vectors may be used as templates for *in vitro* transcription. The IVT vector may include a 5' RNA polymerase promoter, a 5' UTR, a 3' UTR, or a gene of interest flanked by the 5' UTR and 3' UTR, and a 3' polyadenyl cassette containing A nucleotides.

The 3' polyA of RNA is crucial for the nuclear export, RNA stability, and translation efficiency of eukaryotic mRNA. It is known that the 3' polyA sequence shortens over time, and when it becomes sufficiently short, the RNA is enzymatically degraded.

Therefore, even with the aforementioned prior art, there is a need for nucleic acid molecules containing a modified polyadenyl sequence and an OX40L coding sequence, nucleic acid molecules encoding the same, and uses thereof.

### Disclosure of Invention

### Technical Problem

An aspect provides an mRNA molecule containing a coding sequence for a polypeptide containing the extracellular domain of OX40L and a modified polyadenyl sequence.

Another aspect provides a nucleic acid molecule encoding the mRNA molecule.

Another aspect provides a method of amplifying the nucleic acid molecule.

Another aspect provides a method of obtaining RNA.

Another aspect provides a method of obtaining a polypeptide containing the extracellular domain of OX40L.

Another aspect provides a composition for delivering an mRNA containing the mRNA molecule, or a product expressed thereby, to a subject.

Another aspect provides a method of delivering the aforementioned mRNA molecule to a subject.

### Solution to Problem

A first aspect provides an mRNA molecule including a coding sequence for a polypeptide containing the extracellular domain of OX40L and a modified polyadenyl sequence, wherein the modified polyadenyl sequence includes (a) a first sequence including one or more microRNA binding sequences, (b) a second sequence located at 5' of the first sequence and including 20 or fewer consecutive A nucleotides, and (c) a third sequence located at 3' of the first sequence and including 80 or more consecutive A nucleotides.

OX40L is a ligand for OX40. OX40L has structural similarity to TNF and may form cell-bound or secreted trimers. OX40L is present in activated antigen-presenting B and T cells and dendritic cells, vascular endothelial cells, and other non-hematological tissues (e.g., heart, muscle, and pancreas). OX40L is known to interact with OX40R to form a homotrimer. The interaction between OX40L and OX40R is known to exhibit a co-stimulatory effect on OX40R-expressing effector T cells, thereby inducing a more robust cell response through upregulation of cytokine production by T helper cells and inducing increased survival of memory T cells through inhibition of activation-induced cell death. The polypeptide containing the extracellular domain of OX40L may be soluble. The polypeptide containing the extracellular domain of OX40L may be fused with an element promoting formed trimer formation. The element may be a polypeptide promoting trimer formation. The element may be MBL, ferritin, or a fragment thereof. The fragment may be the alpha-helix region of MBL. The alpha-helix region of MBL may be, for example, the sequence of 110^{th} to 129^{th} amino acids of the full-length sequence of MBL. The fragment may be the heavy chain region of ferritin. The heavy chain region may be, for example, the sequence of 15^{th} to 161^{st} amino acids of the full-length sequence of ferritin.

The polypeptide containing the extracellular domain of OX40L may be fused with one or two or more elements. The polypeptide containing the extracellular domain of OX40L may be fused with one or two or more elements through a linker. The linker may be a peptide linker. The peptide linker may be a commonly known short peptide linker. The peptide linker may be a linker including G₄S. The peptide linker may be a linker including G₄S, (G₄S)₂, (G₄S)₃, (G₄S)₄, (G₄S)₅, or (G₄S)₆. The polypeptide containing the extracellular domain of OX40L may include a structure of OX40L extracellular domain-linker-MBL alpha-helix-linker-ferritin, ferritin-linker-MBL alpha-helix-linker-OX40L extracellular domain, MBL-linker-OX40L extracellular domain, or OX40L extracellular domain-linker-MBL.

The polypeptide containing the extracellular domain of OX40L may be a recombinant one including a fusion protein. The sequence encoding the polypeptide containing the extracellular domain of OX40L may be codon-optimized. The codon optimization may vary depending on a host cell into which the nucleic acid molecule is introduced. In addition, the sequence encoding the polypeptide containing the extracellular domain of OX40L may include a degenerate sequence.

In the present disclosure, "polyadenyl sequence," "polyA," or "polyA tail" refers to a region of mRNA containing a plurality of consecutive adenosine monophosphates, which is located downstream, for example, directly downstream (i.e., 3'), of a 3' UTR. The polyA tail may include 10 to 1,000 adenosine monophosphates. For example, the polyA tail may include 10, 20, 30, 40, 50, 60, 70, 80, 90, 100, 110, 120, 130, 140, 150, 160, 170, 190, 200, 210, 220, 230, 240, 250, 260, 270, 280, 290, 300, 350, 400, 450, 500, 600, 700, 800, 900, or 1,000 or more A nucleotides. The polyA tail may include, for example, 50 to 1,000, 60 to 800, 80 to 600, 100 to 500, 50 to 500, 60 to 500, 70 to 500, 80 to 500, 90 to 500, 100 to 500, 50 to 400, 60 to 400, 70 to 400, 80 to 400, 90 to 400, 100 to 400, 50 to 300, 60 to 300, 70 to 300, 80 to 300, 90 to 300, 100 to 300, 50 to 200, 50 to 250, 60 to 200, 70 to 200, 80 to 200, 90 to 200, 100 to 200, 50 to 100, 60 to 100, 70 to 100, 80 to 100, 90 to 100, or 150 to 250 consecutive A nucleotides. In a living body such as a cell or a subject, the polyA tail may serve to protect the mRNA from attack by enzymes, for example, enzymes in the cytoplasm, and to aid in transcription termination, nuclear export of mRNA, and translation.

Polyadenylation is the addition of a polyA sequence to a primary transcript RNA. The polyA sequence is composed of a plurality of adenosine monophosphates. That is, the polyA sequence is a stretch of RNA having only adenine bases. The polyA sequence is important for nuclear export, translation, and stability of the mRNA. The polyA sequence shortens over time, and if it is sufficiently short, the mRNA may be degraded by enzymes.

In the mRNA molecule, the modified polyadenyl sequence may include one or more disruptions in a continuous A sequence, to which one or more microRNA binding sequences are linked. The modified polyadenyl sequence may perform the original function of a polyadenyl sequence to a degree equal or superior to that of an unmodified polyadenyl sequence. The original function may be to aid nuclear export, translation, and stability of mRNA, for example, to enhance them.

In the present disclosure, the term "microRNA" or "miRNA" is used with a meaning known in the art. The microRNA may be a long non-coding RNA of 19 to 25 nucleotides that binds to a nucleic acid molecule to down-regulate gene expression. The microRNA may bind to the 3' UTR of the mRNA. The microRNA may include a sequence complementary to a region of the target mRNA to which it binds. The complementary sequence may be a perfectly or near-perfectly complementary sequence. The down-regulation of gene expression may include being down-regulated by reducing stability of a nucleic acid molecule or inhibiting translation.

The term "microRNA binding sequence" may be any nucleotide sequence with which a microRNA binds or associates. "Binding" may follow conventional Watson-Crick hybridization rules or reflect any stable association between the microRNA and a target sequence at or adjacent to the microRNA binding sequence. The microRNA binding sequence may be a microRNA target sequence, a microRNA sequence, a microRNA seed sequence, or a combination thereof. The microRNA binding sequence may be a known sequence. A microRNA sequence includes a "seed sequence." The seed sequence may be a sequence in a region of the 2^{nd} to 8^{th} positions of a mature microRNA. The seed sequence may have complete Watson-Crick complementarity to the microRNA target sequence. An example of the microRNA sequence may include the 2^{nd} to 8^{th} positions or the 2^{nd} to 7^{th} positions of a mature microRNA. The microRNA sequence includes seven nucleotides (e.g., 2^{nd} to 8^{th} nucleotides of a mature microRNA), and a seed-complementary site of a corresponding miRNA target may be flanked by an adenine (A) opposite position 1 of the microRNA. In addition, the microRNA sequence includes six nucleotides (e.g., 2^{nd} to 7^{th} nucleotides of a mature microRNA), and a seed-complementary site of a corresponding miRNA target may be adjacent to an adenine (A) corresponding to position 1 of the microRNA. The microRNA binding sequence may be natural or artificially created.

In the first sequence of the modified polyadenyl sequence, when the one or more microRNA binding sequences include two or more microRNA binding sequences, the two or more microRNA binding sequences may be identical to or different from each other. In addition, when the microRNA binding sequence includes two or more microRNA binding sequences, the two or more microRNA binding sequences may have microRNA target sequences, microRNA recognition sequences, or seed sequences thereof that are identical to or different from each other. For example, the two or more microRNA binding sequences may be arranged in the form of ABAB, AABB, or ABBA. The two or more microRNA binding sequences may be linked directly to each other without a spacer. In addition, the two or more microRNA binding sequences may be linked to each other through a spacer. The first sequence may be composed of one or more microRNA binding sequences. The spacer may be a modified or unmodified nucleotide sequence.

In the mRNA molecule, the microRNA may be tumor-specific. The microRNA may be up-regulated or down-regulated in tumor cells compared to normal cells. The microRNA binding sequence may inhibit a tumor by binding to a tumor-specific miRNA, a seed sequence thereof, or a miRNA target sequence *in vivo*.

The microRNA may be selected from the group consisting of miR-340, miR-133a, miR-34b, miR-19, miR-200, miR-15, miR-16, miR-1298, miR-663a, miR-449a, miR-138, miR-126, miR-10a, miR-10b, miR-140-3p, miR-140-5p, miR-204, miR-424, miR-34a, miR-193a-3p, miR-455-5p, miR-455-3p, miR-9, miR-10a, miR-148a, miR-488, miR-196a1, miR-182, miR-96, miR-193b, miR-27a, miR-196b, miR-10b, miR-29b2, miR-23a, miR-107, miR-542-3p, miR-93, miR-365a-4, miR-450a, miR-100, miR-105, miR-363, miR-105, miR-106b, miR-15b, miR-21, miR-376c, miR-93, miR-99b, miR-155, miR-33a, miR-876-3p, miR-362-3p, miR-25, let-7i, miR-423-3p, miR-16-2, miR-29a, miR-30d, miR-320, miR-181c, miR-128a, miR-21, let-7d, miR-450b-5p, miR-371-5p, miR-1245, miR-335, miR-492, miR-874, miR-30b, miR-193a-5p, miR-602, miR-346, miR-663, miR-25, miR-219-5p6, miR-184, miR-135a7, miR-584, miR-665, miR-638, miR-503, miR-628-3p, miR-381, miR-78, miR-92b, miR-149, miR-135b, miR-302d, miR-498, miR-766, miR-1389, miR-623, miR-519c-5p, miR-182, miR-494, miR-129-5p10, miR-513-5p, miR-200b, miR-634, miR-654-5p, miR-518b, miR-658, miR-373, miR-30c-2, miR-130a, miR-557, miR-551a, miR-637, miR-518c, miR-525-5p, miR-596, miR-552, miR-625, miR-183, miR-187, miR-544, miR-891a, miR-519e, miR-933, miR-939, miR-214, miR-671-5p, miR-137, miR-92b, miR-525-3p, miR-19a, and miR-409-5p. An example of the microRNA may be down-regulated in tumor cells. The down-regulated miR may be miR-34a. However, differential expression of miR in cancer cells may vary depending on the cancer type.

In the second sequence of the modified polyadenyl sequence, the length of the consecutive A nucleotides may be any length that provides sufficient stability and/or translation to the mRNA obtained when transcribed, given the microRNA binding sequence and the number thereof, and the length of the consecutive A nucleotides of the third sequence. For example, the length of the consecutive A nucleotides of the second sequence may be 1 to 20, 2 to 20, 3 to 20, 4 to 20, 5 to 20, 6 to 20, 7 to 20, 8 to 20, 9 to 20, 10 to 20, 11 to 20, 12 to 20, 13 to 20, 14 to 20, 15 to 20, 16 to 20, 17 to 20, 18 to 20, 19 to 20, 20, 1 to 19, 2 to 19, 3 to 19, 4 to 19, 5 to 19, 6 to 19, 7 to 19, 8 to 19, 9 to 19, 10 to 19, 11 to 19, 12 to 19, 13 to 19, 14 to 19, 15 to 19, 16 to 19, 17 to 19, 18 to 19, 19, 1 to 18, 2 to 18, 3 to 18, 4 to 18, 5 to 18, 6 to 18, 7 to 18, 8 to 18, 9 to 18, 10 to 18, 11 to 18, 12 to 18, 13 to 18, 14 to 18, 15 to 18, 16 to 18, 17 to 18, 18, 1 to 17, 2 to 17, 3 to 17, 4 to 17, 5 to 17, 6 to 17, 7 to 17, 8 to 17, 9 to 17, 10 to 17, 11 to 17, 12 to 17, 13 to 17, 14 to 17, 15 to 17, 16 to 17, 17, 1 to 16, 2 to 16, 3 to 16, 4 to 16, 5 to 16, 6 to 16, 7 to 16, 8 to 16, 9 to 16, 10 to 16, 11 to 16, 12 to 16, 13 to 16, 14 to 16, 15 to 16, 16, 1 to 15, 2 to 15, 3 to 15, 4 to 15, 5 to 15, 6 to 15, 7 to 15, 8 to 15, 9 to 15, 10 to 15, 11 to 15, 12 to 15, 13 to 15, 14 to 15, 15, 1 to 14, 2 to 14, 3 to 14, 4 to 14, 5 to 14, 6 to 14, 7 to 14, 8 to 14, 9 to 14, 10 to 14, 11 to 14, 12 to 14, 13 to 14, 14, 1 to 13, 2 to 13, 3 to 13, 4 to 13, 5 to 13, 6 to 13, 7 to 13, 8 to 13, 9 to 13, 10 to 13, 11 to 13, 12 to 13, 13, 1 to 12, 2 to 12, 3 to 12, 4 to 12, 5 to 12, 6 to 12, 7 to 12, 8 to 12, 9 to 12, 10 to 12, 11 to 12, 12, 1 to 11, 2 to 11, 3 to 11, 4 to 11, 5 to 11, 6 to 11, 7 to 11, 8 to 11, 9 to 11, 10 to 11, 11, 1 to 10, 2 to 10, 3 to 10, 4 to 10, 5 to 10, 6 to 10, 7 to 10, 8 to 10, 9 to 10, 10, 1 to 9, 2 to 9, 3 to 9, 4 to 9, 5 to 9, 6 to 9, 7 to 9, 8 to 9, 9, 1 to 8, 2 to 8, 3 to 8, 4 to 8, 5 to 8, 6 to 8, 7 to 8, 8, 1 to 7, 2 to 7, 3 to 7, 4 to 7, 5 to 7, 6 to 7, 7, 1 to 6, 2 to 6, 3 to 6, 4 to 6, 5 to 6, 6, 1 to 5, 2 to 5, 3 to 5, 4 to 5, 5, 1 to 4, 2 to 4, 3 to 4, 4 to 4, 1 to 3, 2 to 3, 3, 1 to 2, 2, or 1 nucleotide.

In the third sequence of the modified polyadenyl sequence, the length of the consecutive A nucleotides may be any length that provides sufficient stability and/or translation to the mRNA obtained when transcribed, given the microRNA binding sequence and the number thereof, and the length of the consecutive A nucleotides of the second sequence. For example, the length of the consecutive A nucleotides of the third sequence may be 80 to 150, 80 to 140, 80 to 130, 80 to 120, 80 to 110, 90 to 150, 90 to 140, 90 to 130, 90 to 120, or 90 to 110 nucleotides.

In the mRNA molecule, the length of the modified polyadenyl sequence may vary depending on the number of the given microRNA binding sequences. For example, the length of the modified polyadenyl sequence may be increased from the length of the polyadenyl sequence described for the "polyadenyl sequence" by a length corresponding to the number of microRNA binding sequences to be introduced. For example, in a case where the length of the polyadenyl sequence is 10 to 1,000, 20 to 500, 30 to 500, 50 to 100, 80 to 160, 150 to 250, 100 to 300, 40 to 120, or 120 to 200 nucleotides, when 1, 2, 3, 4, and 5 microRNA binding sequences are included, the length of the modified polyadenyl sequence may have a nucleotide sequence of 29 to 1,025, 39 to 525, 49 to 525, 69 to 125, 99 to 185, 169 to 275, 119 to 325, 59 to 145, 139 to 225, 48 to 1,050, 58 to 550, 68 to 550, 88 to 150, 118 to 210, 188 to 300, 138 to 350, 78 to 170, 158 to 250; 67 to 1,075, 77 to 575, 87 to 575, 107 to 175, 137 to 235, 207 to 325, 157 to 375, 97 to 195, 177 to 275, 86 to 1,100, 96 to 600, 106 to 600, 126 to 200, 156 to 260, 226 to 350, 176 to 400, 116 to 220, 196 to 300, 105 to 1,125, 115 to 625, 125 to 625, 145 to 225, 175 to 285, 245 to 375, 195 to 425, 135 to 245, or 215 to 325 nucleotides. The length of the modified polyadenyl sequence is described as an example and is not limited to the aforementioned length.

In the mRNA molecule, the one or more microRNA binding sequences may be located in a region of positions 21 to 39, 21 to 45, 21 to 70, 21 to 95, 21 to 120, 21 to 145, 11 to 39, 11 to 45, 11 to 70, 11 to 95, 11 to 120, or 11 to 145 of the modified polyadenyl sequence.

The mRNA molecule may include an expression control sequence. The expression control sequence may be a translation control sequence or a sequence related to stabilization of RNA. The expression control sequence may be located upstream or downstream of the sequence encoding the polypeptide containing the extracellular domain of OX40L. The expression control sequence may be located downstream of the sequence encoding the polypeptide containing the extracellular domain of OX40L and upstream of the modified polyadenyl sequence.

The expression control sequence may be a Kozak sequence, a ribosome binding sequence, or a UTR. The UTR may be a 5' UTR, a 3' UTR, or a 5' UTR and a 3' UTR. The 5' UTR, the 3' UTR, or the 5' UTR and the 3' UTR may be a sequence that is homologous or heterologous to the sequence encoding the polypeptide containing the extracellular domain of OX40L. The 5' UTR may be a structured UTR. That is, the 5' UTR may have a three-dimensional structure such as a stem-loop structure.

In the mRNA molecule, the 5' UTR is a native human p53 5' UTR or a native human OX40L 5' UTR, and the 3' UTR may be a native human cytochrome P450 2E1 3' UTR.

In the present disclosure, the "5'-untranslated region (5' UTR)" refers to a region of mRNA that is directly upstream (i.e., 5') from a translation initiation codon, that is, the first codon of an mRNA transcript translated by a ribosome. In addition, the "3'-untranslated region (3' UTR)" refers to a region of mRNA that is directly downstream (i.e., 3') from a stop codon that does not encode a polypeptide, that is, a codon of an mRNA transcript signaling translation termination. The 5' UTR or the 3' UTR may be one found in mRNA expressed in any tissue cell. The tissue may be a tissue where cancer is present. The cancer may be a blood cancer or a solid cancer. The tissue may be liver, muscle, brain, breast, colon, lung, pancreas, ovary, skin, or blood. The tissue cell may be, for example, a liver cell, an endothelial cell, a muscle cell, a blood cell, or an adipose tissue cell. The 5' UTR and the 3' UTR may be independently derived from mRNA encoding a protein selected from the group consisting of p53, OX40L, albumin, serum amyloid A, apolipoprotein A/B/E, transferrin, alpha fetoprotein, erythropoietin, factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen. The 5' UTR may be derived from mRNA encoding human p53, human OX40L, or human C3 protein. In addition, the 3' UTR may be derived from mRNA encoding human CYP450 2E1, human OX40L, or human C3 protein. The 3' UTR may be located at 5' of the polyadenyl sequence.

In the present disclosure, an "open reading frame (ORF)" or "a sequence encoding polypeptide" refers to a continuous region of DNA encoding a polypeptide, which begins with an initiation codon, for example, a methionine codon (ATG), and ends with a stop codon, for example, TAA, TAG, or TGA.

In the present disclosure, the term "operatively linked" refers to linkage of nucleotide sequences on a single nucleic acid fragment such that one function is affected by the other. For example, a promoter is operatively linked to a coding sequence (for example, an ORF) if the promoter is capable of affecting expression of the coding sequence (i.e., if the coding sequence is under transcriptional control of the promoter). The coding sequence may be operatively linked to the control sequence in a sense or antisense direction. In the present disclosure, each sequence included in a nucleic acid molecule represents being operatively linked unless otherwise stated.

The expression control sequence may be a sequence that is homologous or heterologous to the sequence encoding the polypeptide containing the extracellular domain of OX40L.

In the mRNA molecule, the sequence encoding the polypeptide containing the extracellular domain of OX40L and the modified polyadenyl sequence may be transcribed under control of a promoter to be produced as a common transcript. The modified polyadenyl sequence may be located at the 3' end of the common transcript.

The mRNA molecule may include one or more modified nucleotides. The modified nucleotide may be a pseudouridine analog. The pseudouridine may be 1-methylpseudouridine. The modified nucleotide may further include 5-methylcytidine. The mRNA molecule may be one in which all Us are substituted with 1-methylpseudouridine.

The mRNA may further include a chain terminating nucleoside. The chain terminating nucleoside may be selected from the group consisting of 3'-deoxyadenosine (cordycepin), 3'-deoxyuridine, 3'-deoxycytosine, 3'-deoxyguanosine, 3'-deoxythymidine, 2', 3'-dideoxynucleoside, 2', 3'-dideoxyadenosine, 2', 3'-dideoxyuridine, 2', 3'-dideoxycytosine, 2', 3'-dideoxyguanosine, 2', 3'-dideoxythymidine, 2'-deoxynucleoside, and -O-methylnucleoside.

In the mRNA molecule, the sequence encoding the polypeptide containing the extracellular domain of OX40L may be codon-optimized.

In addition, the mRNA may include one or more 5'-cap structures. In the present disclosure, the term "cap" refers to a cap structure found at the 5'-end of an mRNA molecule. The cap generally consists of a guanosine nucleotide connected to the mRNA through a 5'-to-5' triphosphate linkage. This guanosine is methylated at the 7^{th} position. The term "conventional 5'-cap" refers to a naturally occurring RNA 5'-cap, for example, 7-methylguanosine (m7G). In the present disclosure, the term "5'-cap" includes 5'-cap analogs that are similar to an RNA cap structure and, when attached, have an ability to stabilize RNA, for example, *in vivo* and/or in a cell. The one or more 5'-cap structures may be selected from the group consisting of 7-methylguanosine (m7G), ARCA, inosine, N1-methyl-guanosine, 2'-fluoro-guanosine, 7-deaza-guanosine, 8-oxo-guanosine, 2-amino-guanosine, LNA-guanosine, and 2-azido-guanosine. In the mRNA, the GA at the 5'-end may have a cap structure of m7(3'OMeG)(5')ppp(5')(2'OMeA). In the mRNA, all Us may be substituted with N1-methyl-pseudouridine. The mRNA may be one in which all Us are substituted with N1-methyl-pseudouridine and the GA at the 5'-end has a cap structure of m7(3'OMeG)(5')ppp(5')(2'OMeA).

The 5'-cap may be Cap 0, Cap 1, or Cap 2. In Cap 0, the riboses of the first and second cap-proximal nucleotides of the mRNA both include a 2'-hydroxyl. In Cap 1, the riboses of the first and second cap-proximal nucleotides of the mRNA include a 2'-methoxy and a 2'-hydroxyl, respectively. In Cap 2, the riboses of the first and second cap-proximal nucleotides of the mRNA both include a 2'-methoxy.

Providing RNA having a 5'-cap or a 5'-cap analog may be achieved by *in vitro* transcription of a DNA template in the presence of the 5'-cap or the 5'-cap analog. In this case, the 5'-cap or the 5'-cap analog may be co-transcriptionally introduced into the RNA strand produced. Alternatively, for example, RNA may be produced by *in vitro* transcription and then capped post-transcriptionally using a capping enzyme, such as a vaccinia virus capping enzyme.

A second aspect provides a method of obtaining a polypeptide containing the extracellular domain of OX40L, the method including translating the aforementioned mRNA molecule.

A third aspect provides a composition for delivering an mRNA molecule containing the aforementioned mRNA molecule as an active ingredient, or a product expressed thereby, to a subject.

A fourth aspect provides a kit for delivering an mRNA molecule containing the aforementioned mRNA molecule as an active ingredient, or a product expressed thereby, to a subject.

In the third and fourth aspects, the composition or the kit may be a pharmaceutical composition or a kit for treating a cancer. The cancer may be a blood cancer or a solid cancer. The blood cancer may be leukemia, malignant lymphoma, multiple myeloma, or aplastic anemia. The solid cancer may be brain tumor, benign astrocytoma, malignant astrocytoma, pituitary adenoma, meningioma, cerebral lymphoma, oligodendroglioma, craniopharyngioma, ependymoma, brainstem tumor, head and neck tumor, laryngeal cancer, oropharyngeal cancer, nasal cavity cancer, nasopharyngeal cancer, salivary gland cancer, hypopharyngeal cancer, thyroid cancer, oral cancer, thoracic tumor, small cell lung cancer, non-small cell lung cancer, thymic cancer, mediastinal tumor, esophageal cancer, breast cancer, male breast cancer, abdominal tumor, stomach cancer, liver cancer, gallbladder cancer, biliary tract cancer, pancreatic cancer, small intestine cancer, colorectal cancer, anal cancer, bladder cancer, kidney cancer, male genital tumor, penile cancer, prostate cancer, female genital tumor, cervical cancer, endometrial cancer, ovarian cancer, uterine sarcoma, vaginal cancer, vulvar cancer, female urethral cancer, or skin cancer.

The composition may be administered to a subject, and RNA (e.g., mRNA) may be translated *in vivo* to produce a polypeptide.

The composition may be contacted with a cell, a tissue, or a subject in an "effective amount".

The effective amount may be determined based on a target tissue, a target cell, a means of administration, physical properties of a polynucleotide (e.g., size and degree of modified nucleoside content), and other components of the composition.

The composition may be administered in combination with other prophylactic or therapeutic compounds. The prophylactic or therapeutic compound may be, for example, an anti-cancer agent. In the present disclosure, the anti-cancer agent may be any known one.

The composition may be administered intramuscularly, subcutaneously, intradermally, nasally, or pulmonarily. The composition may be administered locally to a tissue where cancer is present. The composition may be administered locally to a tissue containing cells in which the level of cancer-specific miRNA is high inside or outside the cells. The composition may be locally administered to a tissue containing cells in which cancer-specific miRNA is up-regulated or down-regulated, inside or outside the cells, as compared to normal cells.

In the present disclosure, the term "active ingredient" refers to the composition or a polynucleotide included therein, for example, RNA including mRNA. The RNA may be, for example, one encoding a polypeptide having anti-cancer activity.

The composition may include one or more pharmaceutically acceptable carriers, diluents, or excipients. The mRNA in the composition may be formulated or complexed with the carrier, diluent, or excipient. The carrier, diluent, or excipient may be one known in the art.

The polynucleotide in the composition may be formulated or complexed with the carrier, diluent, or excipient. The carrier, diluent, or excipient may be one known in the art.

The relative amounts of the active ingredient, pharmaceutically acceptable carrier, diluent, excipient, and/or other additional components included in the composition may vary depending on the identity, size, and/or condition of a subject to be treated and a route of administration. For example, the composition may include the active ingredient in an amount of 0.1 % to 100 %, for example, 0.5 % to 50 %, 1.0 % to 30 %, 5.0 % to 80 %, or 80 % (w/w) or more.

The composition may be formulated as a nanoparticle. The nanoparticle may be one known in the art for delivering a polynucleotide such as mRNA to a cell. For example, the nanoparticle may be a lipid nanoparticle (LNP). The composition may be formulated as a lipid nanoparticle (LNP) or may be one bound to an LNP. The binding includes binding inside or to the surface of the LNP. For example, the composition may be formulated in a lipid-polycation complex or may be one bound to a lipid-polycation complex. The lipid-polycation complex is also referred to as a cationic lipid nanoparticle. The polycation may include MC3, Lipid 319, C12-200, 5A2-SC8, 306Oi10, Moderna Lipid 5, Acuitas A9, SM-102, ALC-0315, Arcturus lipid 2,2(8,8) 4C CH3, Genevant CL1, and/or cationic polypeptides such as poly-lysine, poly-ornithine, and/or poly-arginine. In addition, the composition may be formulated in or bound to a lipid nanoparticle including non-cationic lipids such as distearoylphosphatidylcholine (DSPC), 1,2-distearoyl-sn-glycero-3-phosphocholine (DSPC), dipalmitoyl phosphatidylcholine (DPPC), a sterol such as cholesterol, or dioleoyl phosphatidylethanolamine (DOPE). In addition, the composition may be formulated in or bound to a lipid nanoparticle including a PEG-lipid such as 1,2-dimyristoyl-rac-glycero-3-methoxypolyethylene glycol-2000 (PEG2000-DMG), 2-[(polyethylene glycol)-2000]-N,N-ditetradecylacetamide (ALC-0159), or polyethylene glycol dimethacrylate (PEG-DMA).

A lipid nanoparticle formulation may include a cationic lipid, a phospholipid, a sterol such as cholesterol, a PEG-lipid, or a combination thereof. For example, the lipid nanoparticle formulation may include a cationic lipid, a phospholipid, a sterol such as cholesterol, and a PEG-lipid. Additionally, the lipid nanoparticle may include a PEG-modified lipid, a non-cationic lipid, a sterol, and an ionizable lipid, or a combination thereof. The lipid nanoparticle may include 0.5 to 15 mol% of a PEG-modified lipid, 5 to 25 mol% of a non-cationic lipid, 25 to 55 mol% of a sterol, and 20 to 60 mol% of an ionizable lipid. The PEG-modified lipid may be 1,2-dimyristoyl-sn-glycerol methoxypolyethylene glycol (PEG2000-DMG), the non-cationic lipid may be 1,2-distearoyl-sn-glycero-3-phosphocholine (DSPC), the sterol may be cholesterol, and the ionizable cationic lipid may be Compound 1 having the following structure.

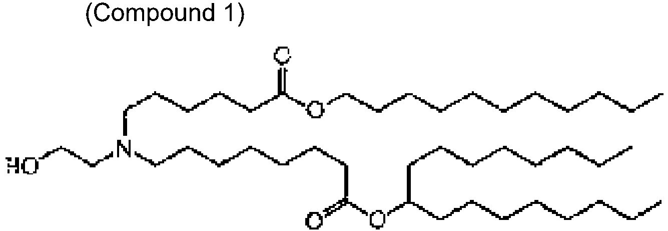

The PEG-modified lipid may be Compound 2 (ALC-0159) having the following structure, the non-cationic lipid may be 1,2-distearoyl-sn-glycero-3-phosphocholine (DSPC), the sterol may be cholesterol, and the ionizable cationic lipid may be Compound 3 (ALC-0315) having the following structure.

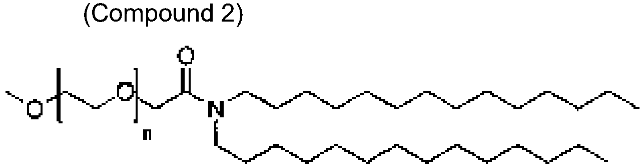

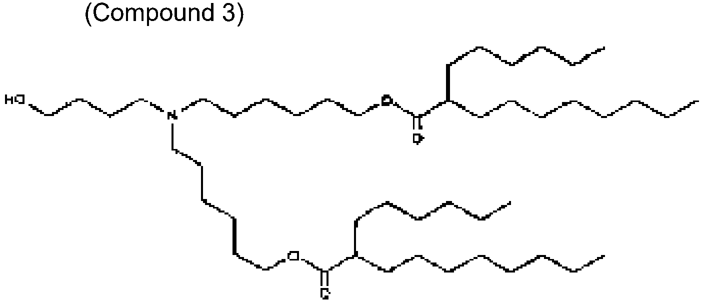

A fifth aspect provides a method of delivering mRNA and a product expressed thereby to a subject, the method including introducing the mRNA into a host cell.

In the method, the host cell may be any cell derived from a subject. The subject may be a mammal including a human. The cell may be, for example, a stem cell, a germ cell, or a somatic cell. In the method, the polynucleotide may be RNA, and the host cell may be a cancer cell, an antigen-presenting cell including a dendritic cell, a monocyte, or a macrophage. The introducing may be locally administering to a tissue where cancer is present. The introducing may be locally administering a nucleic acid molecule or an mRNA molecule to a tissue where cells having a high level of cancer-specific miRNA inside or outside the cells are present. The administration may be local administration of a nucleic acid molecule or an mRNA molecule to a tissue where cells exist in which a cancer-specific miRNA is up-regulated or down-regulated compared to normal cells inside or outside the cells.

The method may include administering the nucleic acid molecule or the mRNA molecule to a subject. The administration may be parenteral or oral. The administration may be intramuscular, subcutaneous, intradermal, nasal, or pulmonary administration. The subject may be a mammal including a human. The administering may be localized administration to a tissue where cancer is present. The administering may be local administration of a polynucleotide, for example, an mRNA molecule, to a tissue where cells having a high level of cancer-specific miRNA inside or outside the cells are present. The administration may be local administration of a polynucleotide, for example, an mRNA molecule, to a tissue where cells in which a cancer-specific miRNA is up-regulated or down-regulated compared to normal cells inside or outside the cells are present.

The method may inhibit cancer cell proliferation by producing the polypeptide containing the extracellular domain of OX40L in a cell. In addition, the method may be for treating a disease in a subject.

A sixth aspect provides a nucleic acid molecule encoding the mRNA.

The nucleic acid molecule may be DNA. In addition, the nucleic acid molecule may include an expression control sequence. The expression control sequence may be a transcriptional control sequence. The expression control sequence may be operatively linked to the sequence encoding the polypeptide containing the extracellular domain of OX40L. The expression control sequence may be located upstream or downstream of the sequence encoding the polypeptide containing the extracellular domain of OX40L. The expression control sequence may be located downstream of the sequence encoding the polypeptide containing the extracellular domain of OX40L and upstream of the modified polyadenyl sequence.

In the present disclosure, the term "transcription" relates to a process in which the genetic code of a DNA sequence is transcribed into RNA. Subsequently, the RNA may be translated into protein. The term "transcription" includes *in vitro* transcription. The term "in vitro transcription" relates to a process in which RNA, particularly mRNA, is synthesized *in vitro* in a cell-free system. Cloning vectors are applied for the generation of transcripts. These cloning vectors are generally also referred to as transcription vectors. The term "vector" includes a transcription vector. RNA is, for example, *in vitro* transcribed RNA (IVT-RNA) and may be obtained by *in vitro* transcription of a DNA template. A promoter for controlling transcription may be any promoter for any RNA polymerase. A DNA template for *in vitro* transcription may be obtained by cloning a nucleic acid, particularly cDNA, and may be introduced into a vector suitable for *in vitro* transcription.

The expression control sequence may be a promoter.

In the present disclosure, the term "promoter" or "promoter region" relates to a DNA sequence upstream (5') of a coding sequence of a gene, and refers to controlling the expression of the coding sequence by providing recognition and binding sites for RNA polymerase. The promoter region may further include recognition or binding sites for additional factors involved in regulating the transcription of the gene. Promoters may control the transcription of prokaryotic or eukaryotic genes. A promoter may be "inducible" and initiate transcription in response to an inducer, or may be "constitutive" if transcription is not controlled by an inducer. In a case where the inducer is not present, transcription of the gene under control of the inducible promoter may occur very little or not at all. In a case where the inducer is present, transcription of the gene under control of the inducible promoter may increase.

The promoter may be one derived from a homologous or heterologous source relative to the sequence encoding the polypeptide containing the extracellular domain of OX40L. The promoter may be one derived from a mammal including a human. The promoter may be one derived from a non-human mammal. The promoter may be one derived from a plant, a microorganism, or a virus. The microorganism may be yeast or bacteria. The bacteria may be Gram-positive or Gram-negative bacteria. The bacteria may be one belonging to the genus *Escherichia*. The promoter may be a T7, T3, or SP6 promoter. The promoter may be one operative in a microorganism of the genus *Escherichia*. The microorganism may be *E. coli*.

In the nucleic acid molecule, the promoter may be a T7 promoter and the microRNA may be microRNA-34a.

The nucleic acid molecule may be a closed circular molecule or a linear molecule.

The nucleic acid molecule may further include one or more selected from the group consisting of (i) a reporter gene, (ii) a selectable marker, and (iii) an origin of replication. The nucleic acid molecule may be a vector. The vector may be a cloning vector or an expression vector.

The nucleic acid molecule may be suitable for *in vitro* transcription of mRNA after being linearized.

In the present disclosure, the term "expression" is used in its general sense and includes the production of RNA or RNA and protein. The expression also includes partial expression of a nucleic acid. In addition, the expression may be transient or stable. With respect to RNA, the term "expression" or "translation" relates to a process in the ribosome of a cell in which a strand of messenger RNA directs the assembly of an amino acid sequence to produce a peptide or protein.

A seventh aspect provides a method of propagating a nucleic acid molecule, the method including (i) providing the aforementioned nucleic acid molecule; and (ii) propagating the nucleic acid molecule in a cell.

In the method, the cell may be a host cell. The term "host cell" refers to any cell into which a foreign nucleic acid may be transformed or transduced. The host cell includes prokaryotic cells (e.g., *E. coli*) or eukaryotic cells (e.g., yeast cells and insect cells). The cell may be an animal, plant, or microbial cell. The animal may be a mammal. The mammal includes humans, mice, rats, hamsters, pigs, and primates. The cell may be derived from a plurality of tissue cells and include primary cells and cell lines. The microorganism may be yeast or bacteria. The bacteria may be Gram-positive or Gram-negative bacteria. The bacteria may be one belonging to the genus *Escherichia*. The cell may be *E. coli*.

In the method, the propagating may include transforming a cell with the nucleic acid molecule, and culturing the transformed cell.

The method may include isolating the nucleic acid molecule from the cell after propagation.

An eighth aspect provides a method of obtaining RNA, including (i) propagating a nucleic acid molecule according to the method of the seventh aspect; and (ii) *in vitro* transcribing the nucleic acid molecule as a template into RNA.

The term "*in vitro* transcription" or "RNA *in vitro* transcription" represents a process in which RNA including mRNA is synthesized in a cell-free system, that is, *in vitro*. Cloning vector DNA including plasmid DNA vectors may be applied as a template for the generation of RNA transcripts. These cloning vectors are generally also referred to as transcription vectors. RNA may be obtained by DNA-dependent *in vitro* transcription of a suitable DNA template. The DNA template may be a linearized plasmid DNA template. A promoter for controlling RNA *in vitro* transcription may be any promoter for DNA-dependent RNA polymerase. The DNA-dependent RNA polymerase may be T7 RNA polymerase, T3 RNA polymerase, SP6 RNA polymerase, or a combination thereof. A DNA template for RNA *in vitro* transcription may be obtained by cloning a nucleic acid and introducing it into a vector for RNA *in vitro* transcription. The DNA may include cDNA corresponding to each RNA to be transcribed *in vitro*. The vector for RNA *in vitro* transcription may be a circular plasmid DNA. The cDNA may be obtained by reverse transcription of mRNA or by chemical synthesis.

A ninth aspect provides a method of obtaining a polypeptide containing the extracellular domain of OX40L, including (i) obtaining mRNA encoding the polypeptide containing the extracellular domain of OX40L according to the method of the eighth aspect; and (ii) translating the mRNA into the polypeptide containing the extracellular domain of OX40L.

The method may include cleaving the nucleic acid molecule before (i).

A tenth aspect provides an RNA molecule obtained by transcribing the nucleic acid molecule of the sixth aspect. The RNA molecule may be an mRNA molecule.

### Advantageous Effects of Invention

The mRNA molecule according to one aspect is relatively stable and exhibits high translation efficiency, and thus may be used to efficiently translate mRNA.

The nucleic acid molecule according to one aspect may be used to produce RNA with enhanced stability.

According to the method of obtaining a polypeptide containing the extracellular domain of OX40L according to another aspect, the polypeptide may be efficiently obtained.

The composition or kit according to another aspect for delivering mRNA or a product expressed thereby to a subject may be used for treating cancer.

According to the method of delivering mRNA to a subject according to one aspect, mRNA may be efficiently delivered.

### Brief Description of Drawings

FIG. 1 is a diagram illustrating a vector obtained by the same process as in Examples 1 and 2.
FIG. 2 is a diagram illustrating a vector including a T7 promoter, a 5' UTR, and a target protein ORF between Hind III and XhoI restriction enzyme sites in the template vector of FIG. 1.
FIG. 3 is a diagram illustrating the vector OX40L-polyA-miR34a for OX40L gene expression, including a 5' UTR, OX40L or a gene for its fusion protein, a 3' UTR, and a modified polyadenyl sequence containing miR34a.

### Best Mode for Carrying out the Invention

Hereinafter, the present disclosure will be described in more detail through examples. However, these examples are provided for illustrative purposes only, and the scope of the present disclosure is not limited to these examples.

### Example 1: Cloning of 3' UTR Sequence

In the present example, a 3' UTR was cloned to increase the expression level and sequence stability of the mRNA-coding gene. The 3' UTR sequence of human cytochrome P450 2E1 was used as the 3' UTR. The 3' UTR was designed to include XhoI and NheI restriction enzyme sequences at the 5'-end and the 3'-end, respectively, to enable recombination with nucleic acids such as the transcribable nucleic acid sequence or the polyadenyl sequence. The transcribable nucleic acid sequence may be an open reading frame (ORF). The 3' UTR sequence may be linked to the 3'-end of the ORF through the XhoI restriction enzyme site at the 5'-end.

A recombinant 3' UTR sequence including the XhoI restriction enzyme sequence, the 3' UTR sequence of human cytochrome P450 2E1, and the NheI restriction enzyme sequence was synthesized by an *in vitro* nucleic acid synthesis method. The obtained recombinant 3' UTR sequence is DNA. After the recombinant 3' UTR sequence and a template vector were each treated and digested with XhoI and NheI restriction enzymes, both sequences were ligated using a DNA ligase. A pUC57-Kan^{R} vector (custom-made) was used as the template vector. The pUC57-Kan^{R} vector is a pUC57 vector including Kanamycin as an antibiotic resistance gene. The antibiotic resistance gene is a selection marker. As a result, a template vector including the recombinant 3' UTR sequence was constructed. Table 1 shows the sequence encoding the 3' UTR sequence of human cytochrome P450 2E1.

**[Table 1]**

| SEQ ID NO: | 3' UTR Sequence |
|---|---|
| 1 | GTGTGTGGAGGACACCCTGAACCCCCCGCTTTCAAACAAGTTTTCA AATTGTTTGAGGTCAGGATTTCTCAAACTGATTCCTTTCTTTGCATA TGAGTATTTGAAAATAAATATTTTCCCAGAATATAAATAAATCATCAC ATGATTATTTTAACTAT |

In Table 1, the sequence of SEQ ID NO: 1 is a wild-type sequence of the 3' UTR of the human cytochrome P450 2E1 gene.

### Example 2: Cloning of consecutive adenosine sequence including miRNA binding sequence

In the present example, in the template vector containing the recombinant 3' UTR prepared in Example 1, a recombinant template vector in which a modified polyadenyl sequence is linked to the 3'-end of the 3' UTR sequence was constructed. The modified polyadenyl sequence is intended to increase the stability and expression level of a gene encoding mRNA.

The modified polyadenyl sequence includes a nucleotide sequence complementary to miRNA-34a in the middle region of an unmodified polyadenyl sequence with a length of 100 nt. Specifically, the modified polyadenyl sequence is one in which the 1^{st} to 22^{nd} sequences of a nucleotide sequence complementary to miRNA-34a are introduced between the 20^{th} and 21^{st} positions of the unmodified polyadenyl sequence.

In addition, the modified polyadenyl sequence was configured to include NheI and EcoRI restriction enzyme sequences linked to the 5'-end and the 3'-end, respectively, to enable use for recombination. A recombinant modified polyadenyl sequence including the NheI restriction enzyme sequence, the modified polyadenyl sequence, and the EcoRI restriction enzyme sequence was synthesized by an *in vitro* nucleic acid synthesis method. The obtained recombinant sequence is DNA.

After the obtained recombinant modified polyadenyl sequence and the template vector containing the recombinant 3' UTR prepared in Example 1 were each treated and digested with NheI and EcoRI restriction enzymes, both sequences were ligated using a DNA ligase to construct a template vector containing the 3' UTR-modified polyadenyl sequence. Table 2 shows the nucleotide sequence of the modified polyadenyl sequence.

**[Table 2]**

| SEQ ID NO: | PolyA Tail Sequence |
|---|---|
| 2 | AAAAAAAAAAAAAAAAAAAAACAACCAGCTAAGACACTGCCAAAAAA AAAAAAAAAAAAAAAAAAAAAAAAAAAAAAAAAAAAAAAAAAAAAAAA AAAAAAAAAAAAAAAAAAAAAAAAAAAAAAAAAAAAAAAAAAAAAAA |

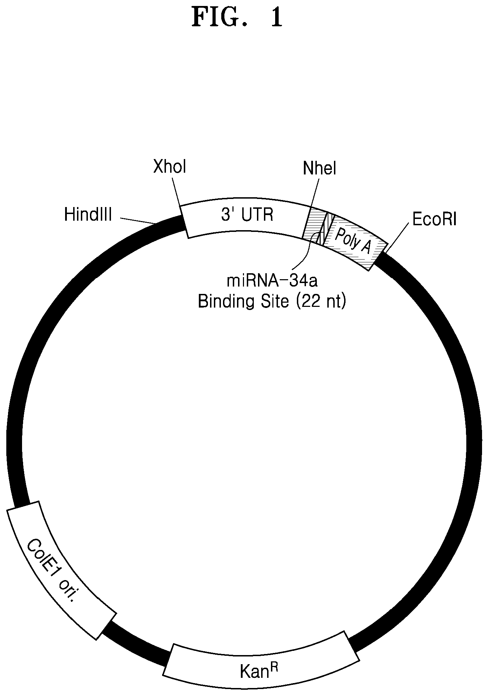

In the sequence described in Table 2, the underlined nucleotides are a sequence complementary to miRNA-34a, that is, the miRNA-34a binding sequence. FIG. 1 illustrates a vector obtained by the same processes as in Examples 1 and 2. As shown in FIG. 1, the vector includes a 3' UTR linked to the 5' of a modified polyadenyl sequence including a miRNA-34a binding sequence in the region of the 21^{st} to 42^{nd} positions of the nucleotide sequence thereof. Because the vector includes HindIII and XhoI restriction enzyme recognition sites upstream of the 3' UTR, the vector may be used to introduce a target protein ORF linked to the 3'-end of a 5' UTR. That is, the vector may be a platform vector used for cloning an ORF. The 5' UTR-ORF is linked to a T7 promoter upstream thereof. The vector also includes an antibiotic resistance marker (Kan^{R}) and an *E. coli* origin of replication (ColE1 ori).

The nucleotide sequence of the template vector as shown in FIG. 1 is shown in Table 3.

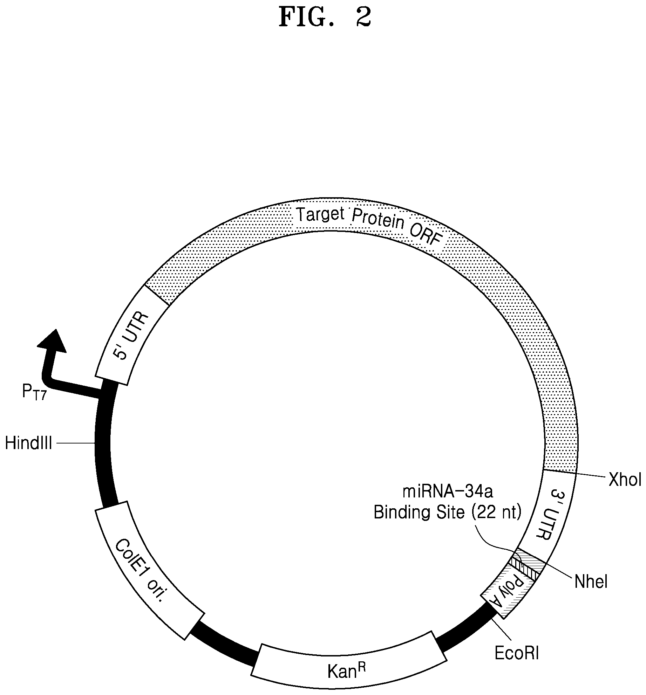

FIG. 2 illustrates a vector including a T7 promoter, a 5' UTR, and a target protein ORF between the HindIII and XhoI restriction enzyme sites in the template vector of FIG. 1.

**[Table 3]**

| SEQ ID NO: | Nucleotide Sequence |
|---|---|
| 3 | AAGCTTCTCGAGgtgtgtggaggacaccctgaaccccccgctttcaaacaagttttcaaattgttt gaggtcaggatttctcaaactgattcctttctttgcatatgagtatttgaaaataaatattttcccagaatataa ataaatcatcacatgattattttaactatgctagcAAAAAAAAAAAAAAAAAAaaacaaccag |
| | ctaagacactgccaAAAAAAAAAAAAAAAAAAAAAAAAAAAAAAAAAAAA AAAAAAAAAAAAAAAAAAAAAAAAAAAAAAAAAAAAAAAAAAAAAAAAAA AAAAAAAAAAAAAAGAAGAGCGAATTCTCAAGACGATAGTTACCGGATAAG GCGCAGCGGTCGGGCTGAACGGGGGGTTCGTGCACACAGCCCAGCT TGGAGCGAACGACCTACACCGAACTGAGATACCTACAGCGTGAGCTA TGAGAAAGCGCCACGCTTCCCGAAGGGAGAAAGGCGGACAGGTATC CGGTAAGCGGCAGGGTCGGAACAGGAGAGCGCACGAGGGAGCTTCC AGGGGGAAACGCCTGGTATCTTTATAGTCCTGTCGGGTTTCGCCACC TCTGACTTGAGCGTCGATTTTTGTGATGCTCGTCAGGGGGGCGGAGC CTATGGAAAAACGCCAGCAACGCGGCCTTTTTACGGTTCCTGGCCTTT TGCTGGCCTTTTGCGAGCCTTTTGCTCACATGTTCTTTCCTGCGTTATC CCCTGATTCTGTGGATAACCGTATTACCGCCTTTGAGTGAGCTGATAC CGCTCGCCGCAGCCGAACGACCGAGCGCAGCGAGTCAGTGAGCGAG GAAGCGGCCCAATACGCAAACCGCCTCTCCCCGCGCGTTGGCCGATT CATTAATGCAGCTGGCACGACAGGTTTCCCGACTGGAAAGCGGGCAG TGAGCGCAACGCAATTAATGTGAGTTAGCTCACTCATTAGGCACCCCA GGCTTTACACTTTATGCTTCCGGCTCGTATGTTGTGTGGAATTGTGAG CGGATAACAATTTCACACAGGAAACAGCTATGACCATGATTACGCC |
| 4 | gtgtgtggaggacaccctgaaccccccgctttcaaacaagttttcaaattgtttgaggtcaggatttctcaa actgattcctttctttgcatatgagtatttgaaaataaatattttcccagaatataaataaatcatcacatgatt attttaactatAAAAAAAAAAAAAAAAAAAAacaaccagctaagacactgccaGCCACC ACTTCAAGAACTCTGTAGCACCGCCTACATACCTCGCTCTGCTAATCC TGTTACCAGTGGCTGCTGCCAGTGGCGATAAGTCGTGTCTTACCGGG TTGGACTCAAGACGATAGTTACCGGATAAGGCGCAGCGGTCGGGCTG AACGGGGGGTTCGTGCACACAGCCCAGCTTGGAGCGAACGACCTAC ACCGAACTGAGATACCTACAGCGTGAGCTATGAGAAAGCGCCACGCT TCCCGAAGGGAGAAAGGCGGACAGGTATCCGGTAAGCGGCAGGGTC GGAACAGGAGAGCGCACGAGGGAGCTTCCAGGGGGAAACGCCTGGT ATCTTTATAGTCCTGTCGGGTTTCGCCACCTCTGACTTGAGCGTCGAT TTTTGTGATGCTCGTCAGGGGGGCGGAGCCTATGGAAAAACGCCAGC AACGCGGCCTTTTTACGGTTCCTGGCCTTTTGCTGGCCTTTTGCGAGC |
| | CTTTTGCTCACATGTTCTTTCCTGCGTTATCCCCTGATTCTGTGGATAA CCGTATTACCGCCTTTGAGTGAGCTGATACCGCTCGCCGCAGCCGAA CGACCGAGCGCAGCGAGTCAGTGAGCGAGGAAGCGGCCCAATACGC AAACCGCCTCTCCCCGCGCGTTGGCCGATTCATTAATGCAGCTGGCA CGACAGGTTTCCCGACTGGAAAGCGGGCAGTGAGCGCAACGCAATTA ATGTGAGTTAGCTCACTCATTAGGCACCCCAGGCTTTACACTTTATGC TTCCGGCTCGTATGTTGTGTGGAATTGTGAGCGGATAACAATTTCACA CAGGAAACAGCTATGACCATGATTACGCC |

Among the sequence of the template vector of SEQ ID NO: 3 described in Table 3, the underlined nucleotides in uppercase are a HindIII restriction enzyme recognition sequence. Among the sequence of the template vector of SEQ ID NO: 3 described in Table 3, the bolded nucleotides in uppercase are a XhoI restriction enzyme recognition sequence.

Among the sequence of the template vector of SEQ ID NO: 3 described in Table 3, the nucleotides indicated in lowercase are a wild-type sequence of the human cytochrome P450 2E1 3' UTR (SEQ ID NO: 1).

Among the sequence of the template vector of SEQ ID NO: 3 described in Table 3, the bolded nucleotides in lowercase are a NheI restriction enzyme recognition sequence.

Among the sequence of the template vector of SEQ ID NO: 3 described in Table 3, the underlined nucleotides in lowercase are a miRNA-34a binding sequence complementary to miRNA-34a.

Among the sequence of the template vector of SEQ ID NO: 3 described in Table 3, the italicized nucleotides in uppercase are an EcoRI restriction enzyme recognition sequence.

SEQ ID NO: 4 described in Table 3 is a sequence excluding the restriction enzyme recognition sites from SEQ ID NO: 3.

### Example 3: Construction of OX40L mRNA transcription vector

In the present example, a template vector to be used for OX40L mRNA transcription was constructed using the vector platform shown in FIG. 1 prepared in Example 2.

A recombinant gene including a 5' restriction enzyme recognition site, a T7 promoter sequence, a 5' UTR, an open reading frame, and a 3' restriction enzyme recognition site was synthesized by an *in vitro* nucleic acid synthesis method. The 5' restriction enzyme recognition site and the 3' restriction enzyme recognition site are HindIII and XhoI restriction enzyme sequences, respectively. The open reading frame (ORF) is a nucleotide sequence encoding each of native OX40L, a fusion protein of OX40L-MBL alpha-helix region-ferritin, or a fusion protein of MBL-OX40L. The heavy chain region of native ferritin was used as the ferritin. MBL represents mannan-binding lectin, and a sequence from which a carbohydrate recognition domain was removed was used.

As a result, a recombinant sequence in which the HindIII recognition site, the T7 promoter sequence, the 5' UTR, each of the aforementioned ORFs, and the XhoI recognition site are linked was obtained. The fusion protein includes a linker sequence between each fusion partner. The linker sequence is G4S, that is, a GGGGS sequence.

Next, after the obtained recombinant sequence and the template vector of FIG. 1 prepared in Example 2 were each treated and digested with restriction enzymes HindIII and XhoI, both sequences were ligated using a DNA ligase.

As a result, a transcription vector including the 5' UTR, each of the aforementioned ORFs, the cytochrome P450 2E1 3' UTR, and the modified polyadenyl sequence was constructed. Table 4 shows the nucleotides of the recombinant sequence in which the T7 promoter sequence, the 5' UTR, the OX40L ORF, the ORF of the fusion protein of OX40L-MBL alpha-helix region-ferritin, or the ORF of the fusion protein of MBL-OX40L are linked. The HindIII recognition site and the XhoI recognition site sequences are not included in the 5' UTR and the ORF, respectively, and were used only for cloning.

**[Table 4]**

| SEQ ID NO: | Region | Nucleotide Sequence |
|---|---|---|
| 5 | T7 Promoter | TAATACGACTCACTATA |
| 6 | 5' UTR | AGTGCTCCCCATCCAACTAAACTGTCCCTCTGTCCG AACTAAACTGCACTGTCCCAGCACC |
| 11 | Signal Sequence | ATGACAAGGTTGACGGTCCTGGCATTGCTTGCCGGG CTTTTGGCGAGTTCTCGGGCG |
| 12 | Extracellular Domain of OX40L | AGCAGTTCACCTGCAAAGGACCCCCCAATCCAGCGA CTGAGAGGCGCAGTCACCCGGTGTGAAGATGGACA GCTTTTTATATCATCTTACAAGAATGAGTATCAGACT ATGGAGGTTCAAAATAACTCCGTGGTTATAAAGTGC GACGGGCTTTACATCATCTATCTGAAGGGGTCCTTTT TCCAGGAGGTCAAGATAGATTTGCACTTCAGGGAAG ACCATAACCCCATTTCTATTCCTATGCTTAATGACGG ACGACGGATCGTATTTACTGTTGTGGCTTCTCTCGCT TTTAAGGACAAGGTCTACCTTACCGTGAATGCCCCA GACACACTTTGCGAGCACCTCCAGATCAACGACGGA GAGCTTATCGTTGTTCAACTTACACCAGGTTATTGCG CTCCAGAGGGGAGCTATCACTCCACCGTCAACCAG GTACCCCTT |
| 13 | G4S linker*2 | GGTGGAGGCGGTTCAGGCGGAGGTGGCTCT |
| 14 | Alpha-helix of MBL | AGCGAGAGAAAGGCCCTTCAGACAGAAATGGCACG CATAAAAAAATGGCTTACATTCAGT |
| 26 | G4S linker | GGTGGAGGCGGTTCA |
| 15 | Ferritin | CAGGACTCCGAAGCAGCAATAAACCGACAAATAAAT CTGGAGTTGTACGCATCCTACGTTTACCTGTCCATGT CTTACTACTTCGATAGAGACGATGTCGCCCTCAAAAA CTTCGCCAAGTATTTCCTGCATCAGTCCCATGAAGA GAGAGAGCACGCAGAGAAACTGATGAAACTTCAGAA CCAACGGGGGGGCAGGATCTTTCTGCAAGACATTAA GAAGCCTGATTGCGATGACTGGGAATCTGGACTTAA TGCCATGGAGTGTGCCCTGCACCTCGAAAAAAATGT GAACCAGAGCCTGTTGGAATTGCATAAGCTGGCAAC CGATAAGAACGATCCACATCTTTGCGATTTCATCGAA ACCCACTATCTCAATGAACAAGTGAAGGCAATTAAG |
| | | GAGCTGGGCGATCATGTAACAAATTTGCGAAAAATG GGTGCC |
| 5 | T7 Promoter | TAATACGACTCACTATA |
| 6 | 5' UTR | AGTGCTCCCCATCCAACTAAACTGTCCCTCTGTCCG AACTAAACTGCACTGTCCCAGCACC |
| 11 | Signal Sequence | ATGACAAGGTTGACGGTCCTGGCATTGCTTGCCGGG CTTTTGGCGAGTTCTCGGGCG |
| 16 | MBL | TCTGGCTCTCAGACTTGTGAAGATACCCTTAAGACTT GCAGTGTCATAGCCTGTGGGCGAGACGGGCGCGAT GGACCCAAGGGCGAAAAAGGAGAACCAGGGCAAGG GCTCAGAGGACTGCAAGGTCCACCTGGTAAATTGGG GCCACCTGGAAGCGTGGGGTCCCCAGGAAGTCCAG GCCCAAAAGGTCAGAAAGGGGACCACGGCGATAAT CGAGCCATTGAGGAAAAACTCGCAAATATGGAGGCC GAAATTAGGATTCTGAAGAGTAAGTTGCAGCTGACC AACAAGCTCCATGCTTTTAGCATGGGC |
| 13 | G4S linker*2 | GGTGGAGGCGGTTCAGGCGGAGGTGGCTCT |
| 12 | Extracellular Domain of OX40L | AGCAGTTCACCTGCAAAGGACCCCCCAATCCAGCGA CTGAGAGGCGCAGTCACCCGGTGTGAAGATGGACA GCTTTTTATATCATCTTACAAGAATGAGTATCAGACT ATGGAGGTTCAAAATAACTCCGTGGTTATAAAGTGC GACGGGCTTTACATCATCTATCTGAAGGGGTCCTTTT TCCAGGAGGTCAAGATAGATTTGCACTTCAGGGAAG ACCATAACCCCATTTCTATTCCTATGCTTAATGACGG ACGACGGATCGTATTTACTGTTGTGGCTTCTCTCGCT TTTAAGGACAAGGTCTACCTTACCGTGAATGCCCCA GACACACTTTGCGAGCACCTCCAGATCAACGACGGA GAGCTTATCGTTGTTCAACTTACACCAGGTTATTGCG CTCCAGAGGGGAGCTATCACTCCACCGTCAACCAG GTACCCCTT |

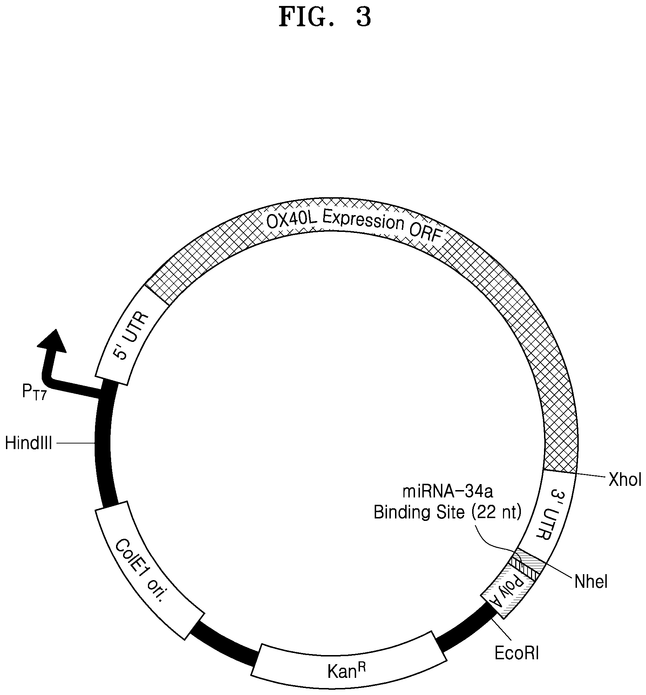

FIG. 3 illustrates a vector OX40L-polyA-miR34a for expressing OX40L or a fusion protein gene thereof, which includes a 5' UTR, an OX40L gene or a fusion protein gene of OX40L, a 3' UTR, and a modified polyadenyl sequence including miR34a. As shown in FIG. 3, the template vector includes a promoter-5' UTR-OX40L ORF-CYP450 2E1 3' UTR-modified polyadenyl sequence containing a miRNA-34a binding sequence. In addition, the template vector includes ColE1 ori and a Kan^{R} gene. Table 5 shows the nucleotide sequence of each element among products obtained by *in vitro* transcription of the obtained transcription vector. Table 6 shows the amino acid sequence encoded by each ORF included in the obtained transcription vector.

**[Table 5]**

| Mate rial | SEQ ID NO: | Region | mRNA Sequence |
|---|---|---|---|
| 1 | 7 | 5' UTR | AGUGCUCCCCAUCCAACUAAACUGUCCCUCUGUCC GAACUAAACUGCACUGUCCCAGCACC |
| | 17 | Signal Sequence | AUGACAAGGUUGACGGUCCUGGCAUUGCUUGCCGG GCUUUUGGCGAGUUCUCGGGCG |
| | 18 | Extracellular Domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUUA AUGACGGACGACGGAUCGUAUUUACUGUUGUGGCU UCUCUCGCUUUUAAGGACAAGGUCUACCUUACCGU GAAUGCCCCAGACACACUUUGCGAGCACCUCCAGA UCAACGACGGAGAGCUUAUCGUUGUUCAACUUACA CCAGGUUAUUGCGCUCCAGAGGGGAGCUAUCACUC CACCGUCAACCAGGUACCCCUU |
| | 19 | G4S linker*2 | GGUGGAGGCGGUUCAGGCGGAGGUGGCUCU |
| | 20 | Alpha-helix of MBL | AGCGAGAGAAAGGCCCUUCAGACAGAAAUGGCACG CAUAAAAAAAUGGCUUACAUUCAGU |
| | 27 | G4S Linker | GGUGGAGGCGGUUCA |
| | 21 | Ferritin | CAGGACUCCGAAGCAGCAAUAAACCGACAAAUAAAU CUGGAGUUGUACGCAUCCUACGUUUACCUGUCCAU GUCUUACUACUUCGAUAGAGACGAUGUCGCCCUCA AAAACUUCGCCAAGUAUUUCCUGCAUCAGUCCCAUG AAGAGAGAGAGCACGCAGAGAAACUGAUGAAACUUC AGAACCAACGGGGGGGCAGGAUCUUUCUGCAAGAC AUUAAGAAGCCUGAUUGCGAUGACUGGGAAUCUGG ACUUAAUGCCAUGGAGUGUGCCCUGCACCUCGAAA AAAAUGUGAACCAGAGCCUGUUGGAAUUGCAUAAG CUGGCAACCGAUAAGAACGAUCCACAUCUUUGCGA UUUCAUCGAAACCCACUAUCUCAAUGAACAAGUGAA GGCAAUUAAGGAGCUGGGCGAUCAUGUAACAAAUU UGCGAAAAAUGGGUGCC |
| | 22 | 3' UTR | CUCGAGGUGUGUGGAGGACACCCUGAACCCCCCGC UUUCAAACAAGUUUUCAAAUUGUUUGAGGUCAGGA UUUCUCAAACUGAUUCCUUUCUUUGCAUAUGAGUA UUUGAAAAUAAAUAUUUUCCCAGAAUAUAAAUAAAU CAUCACAUGAUUAUUUUAACUAUGCUAGC |
| | 8 | PolyA Tail | AAAAAAAAAAAAAAAAAAAAACAACCAGCUAAGACAC UGCCAAAAAAAAAAAAAAAAAAAAAAAAAAAAAAAAAA AAAAAAAAAAAAAAAAAAAAAAAAAAAAAAAAAAAAAA AAAAAAAAAAAAAAAAAAAAAAAAAAAAAAGAAGAGC GAAUU |
| 2 | 7 | 5' UTR | AGUGCUCCCCAUCCAACUAAACUGUCCCUCUGUCC GAACUAAACUGCACUGUCCCAGCACC |
| | 17 | Signal Sequence | AUGACAAGGUUGACGGUCCUGGCAUUGCUUGCCGG GCUUUUGGCGAGUUCUCGGGCG |
| | 23 | MBL | UCUGGCUCUCAGACUUGUGAAGAUACCCUUAAGAC UUGCAGUGUCAUAGCCUGUGGGCGAGACGGGCGC GAUGGACCCAAGGGCGAAAAAGGAGAACCAGGGCA AGGGCUCAGAGGACUGCAAGGUCCACCUGGUAAAU UGGGGCCACCUGGAAGCGUGGGGUCCCCAGGAAG UCCAGGCCCAAAAGGUCAGAAAGGGGACCACGGCG AUAAUCGAGCCAUUGAGGAAAAACUCGCAAAUAUGG AGGCCGAAAUUAGGAUUCUGAAGAGUAAGUUGCAG CUGACCAACAAGCUCCAUGCUUUUAGCAUGGGC |
| | 19 | G4S linker*2 | GGUGGAGGCGGUUCAGGCGGAGGUGGCUCU |
| | 18 | Extracellular Domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUUA AUGACGGACGACGGAUCGUAUUUACUGUUGUGGCU UCUCUCGCUUUUAAGGACAAGGUCUACCUUACCGU GAAUGCCCCAGACACACUUUGCGAGCACCUCCAGA UCAACGACGGAGAGCUUAUCGUUGUUCAACUUACA CCAGGUUAUUGCGCUCCAGAGGGGAGCUAUCACUC CACCGUCAACCAGGUACCCCUU |
| | 22 | 3' UTR | CUCGAGGUGUGUGGAGGACACCCUGAACCCCCCGC UUUCAAACAAGUUUUCAAAUUGUUUGAGGUCAGGA UUUCUCAAACUGAUUCCUUUCUUUGCAUAUGAGUA UUUGAAAAUAAAUAUUUUCCCAGAAUAUAAAUAAAU CAUCACAUGAUUAUUUUAACUAUGCUAGC |
| | 8 | PolyA Tail | AAAAAAAAAAAAAAAAAAAAAAAACAACCAGCUAAGACAC UGCCAAAAAAAAAAAAAAAAAAAAAAAAAAAAAAAAAAAA AAAAAAAAAAAAAAAAAAAAAAAAAAAAAAAAAAAAAAAA |
| | | | AAAAAAAAAAAAAAAAAAAAAAAAAAAAAAGAAGAGC GAAUU |
| 3 | 7 | 5' UTR | AGUGCUCCCCAUCCAACUAAACUGUCCCUCUGUCC GAACUAAACUGCACUGUCCCAGCACC |
| | 17 | Signal Sequence | AUGACAAGGUUGACGGUCCUGGCAUUGCUUGCCGG GCUUUUGGCGAGUUCUCGGGCG |
| | 18 | Extracellular Domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUUA AUGACGGACGACGGAUCGUAUUUACUGUUGUGGCU UCUCUCGCUUUUAAGGACAAGGUCUACCUUACCGU GAAUGCCCCAGACACACUUUGCGAGCACCUCCAGA UCAACGACGGAGAGCUUAUCGUUGUUCAACUUACA CCAGGUUAUUGCGCUCCAGAGGGGAGCUAUCACUC CACCGUCAACCAGGUACCCCUU |
| | 19 | G4S linker*2 | GGUGGAGGCGGUUCAGGCGGAGGUGGCUCU |
| | 20 | Alpha-helix of MBL | AGCGAGAGAAAGGCCCUUCAGACAGAAAUGGCACG CAUAAAAAAAUGGCUUACAUUCAGU |
| | 27 | G4S linker | GGUGGAGGCGGUUCA |
| | 21 | Ferritin | CAGGACUCCGAAGCAGCAAUAAACCGACAAAUAAAU CUGGAGUUGUACGCAUCCUACGUUUACCUGUCCAU GUCUUACUACUUCGAUAGAGACGAUGUCGCCCUCA AAAACUUCGCCAAGUAUUUCCUGCAUCAGUCCCAUG AAGAGAGAGAGCACGCAGAGAAACUGAUGAAACUUC AGAACCAACGGGGGGGCAGGAUCUUUCUGCAAGAC AUUAAGAAGCCUGAUUGCGAUGACUGGGAAUCUGG |
| | | | ACUUAAUGCCAUGGAGUGUGCCCUGCACCUCGAAA AAAAUGUGAACCAGAGCCUGUUGGAAUUGCAUAAG CUGGCAACCGAUAAGAACGAUCCACAUCUUUGCGA UUUCAUCGAAACCCACUAUCUCAAUGAACAAGUGAA GGCAAUUAAGGAGCUGGGCGAUCAUGUAACAAAUU UGCGAAAAAUGGGUGCC |
| | 9 | 3' UTR | GUGUGUGGAGGACACCCUGAACCCCCGCUUUCAA ACAAGUUUCAAAUUGUUUGAGGUCAGGAUUUCUC AAACUGAUUCCUUUCUUUGCAUAUGAGUAUUUGAAA AUAAAUAUUUCCCAGAAUAUAAAUAAAUCAUCACA UGAUUAUUUAACUAU |
| | 10 | PolyA Tail | AAAAAAAAAAAAAAAAAAAAAAAACAACCAGCUAAGACAC UGCCAAAAAAAAAAAAAAAAAAAAAAAAAAAAAAAAAAAAA AAAAAAAAAAAAAAAAAAAAAAAAAAAAAAAAAAAAAAAAA AAAAAAAAAAAAAAAAAAAAAAAAAAAAAAA |
| 4 | 7 | 5' UTR | AGUGCUCCCCAUCCAACUAAACUGUCCCUCUGUCC GAACUAAACUGCACUGUCCCAGCACC |
| | 17 | Signal Sequence | AUGACAAGGUUGACGGUCCUGGCAUUGCUUGCCGG GCUUUUGGCGAGUUCUCGGGCG |
| | 23 | MBL | UCUGGCUCUCAGACUUGUGAAGAUACCCUUAAGAC UUGCAGUGUCAUAGCCUGUGGGCGAGACGGGCGC GAUGGACCCAAGGGCGAAAAAGGAGAACCAGGGCA AGGGCUCAGAGGACUGCAAGGUCCACCUGGUAAAU UGGGGCCACCUGGAAGCGUGGGGUCCCCAGGAAG UCCAGGCCCAAAAGGUCAGAAAGGGGACCACGGCG AUAAUCGAGCCAUUGAGGAAAAACUCGCAAAUAUGG AGGCCGAAAUUAGGAUUCUGAAGAGUAAGUUGCAG CUGACCAACAAGCUCCAUGCUUUUAGCAUGGGC |
| | 19 | G4S linker*2 | GGUGGAGGCGGUUCAGGCGGAGGUGGCUCU |
| | 18 | Extracellular Domain of OX40L | AGCAGUUCACCUGCAAAGGACCCCCCAAUCCAGCG ACUGAGAGGCGCAGUCACCCGGUGUGAAGAUGGAC AGCUUUUUAUAUCAUCUUACAAGAAUGAGUAUCAGA CUAUGGAGGUUCAAAAUAACUCCGUGGUUAUAAAG UGCGACGGGCUUUACAUCAUCUAUCUGAAGGGGUC CUUUUUCCAGGAGGUCAAGAUAGAUUUGCACUUCA GGGAAGACCAUAACCCCAUUUCUAUUCCUAUGCUUA AUGACGGACGACGGAUCGUAUUUACUGUUGUGGCU UCUCUCGCUUUUAAGGACAAGGUCUACCUUACCGU GAAUGCCCCAGACACACUUUGCGAGCACCUCCAGA UCAACGACGGAGAGCUUAUCGUUGUUCAACUUACA CCAGGUUAUUGCGCUCCAGAGGGGAGCUAUCACUC CACCGUCAACCAGGUACCCCUU |
| | 9 | 3' UTR | GUGUGUGGAGGACACCCUGAACCCCCGCUUUCAA ACAAGUUUCAAAUUGUUUGAGGUCAGGAUUUCUC AAACUGAUUCCUUUCUUUGCAUAUGAGUAUUUGAAA AUAAAUAUUUCCCAGAAUAUAAAUAAAUCAUCACA UGAUUAUUUAACUAU |
| | 10 | PolyA Tail | AAAAAAAAAAAAAAAAAAAAAAAACAACCAGCUAAGACAC UGCCAAAAAAAAAAAAAAAAAAAAAAAAAAAAAAAAAAAAA AAAAAAAAAAAAAAAAAAAAAAAAAAAAAAAAAAAAAAAAA AAAAAAAAAAAAAAAAAAAAAAAAAAAAAAA |

In Table 5, the extracellular domain of OX40L is, for example, the OX40 receptor binding domain. In Table 5, the G4S linker*2 represents G4SG4S. Among the 3' UTR region of SEQ ID NO: 27 described in Table 5, the underlined nucleotides are a XhoI restriction enzyme recognition sequence.

Among the 3' UTR region of SEQ ID NO: 27 described in Table 5, the bolded nucleotides are a NheI restriction enzyme recognition sequence.

Among the polyA tail region of SEQ ID NO: 11 described in Table 5, the underlined nucleotides are a SapI restriction enzyme recognition sequence.

Among the polyA tail region of SEQ ID NO: 11 described in Table 5, the bolded nucleotides are a part of an EcoRI restriction enzyme recognition sequence.

The sequences of materials 3 and 4 described in Table 5 represent sequences excluding the restriction enzyme recognition sites shown in materials 1 and 2.

OX40 ligand (OX40L) and OX40 are typical members of the tumor necrosis factor ligand family and the tumor necrosis factor receptor family, respectively. OX40L and OX40 are involved in costimulation and differentiation of T cells. Like other tumor necrosis factor ligands, OX40L is a type II transmembrane protein. Soluble human OX40L is assembled to form a trimer and binds to OX40, but the soluble human OX40L is inactive if not assembled. When transcribed and translated, Materials 1 and 3 are fusion proteins having a structure of OX40L extracellular domain-G4S linker*2-MBL alpha-helix-G4S linker-ferritin. That is, the fusion protein is one in which the extracellular domain of soluble OX40L is linked to each of the alpha-helix of MBL and ferritin through a linker. The fusion protein constitutes a unit in the form of a trimer in which three fusion proteins are assembled, and these units may bind to each other to form an oligomer, for example, an oligomer consisting of eight units. These oligomers may bind to and activate OX40. This may facilitate the interaction between the extracellular domain of OX40L and OX40 more effectively than when soluble OX40L is expressed alone. In the fusion protein, the alpha-helix of MBL may promote trimer unit formation of the fusion protein. In addition, in the fusion protein, ferritin may promote oligomer formation by binding the formed trimer units of the fusion protein to each other. The oligomer may have a cage-like form. In the fusion protein, the extracellular domain of OX40L may be any polypeptide including the extracellular domain of OX40L, for example, full-length OX40L. However, the claims should not be interpreted as being limited to these specific mechanisms.

Materials 2 and 4 are fusion proteins having a structure of MBL-G4S linker*2-OX40L extracellular domain when transcribed and translated. That is, the fusion protein is one in which the extracellular domain of soluble OX40L is linked to MBL through a linker. The fusion protein constitutes a unit in the form of a trimer in which three fusion proteins are assembled, and these units may bind to each other to form an oligomer, for example, an oligomer consisting of one, or two to four units, or more of the trimers. These oligomers may bind to and activate OX40. This may facilitate the interaction between the extracellular domain of OX40L and OX40 more effectively than when soluble OX40L is expressed alone. In the fusion protein, MBL may promote trimer unit formation of the fusion protein. In addition, in the fusion protein, MBL may promote oligomer formation by binding the formed trimer units of the fusion protein to each other. The oligomer may have a bouquet-like structure. In the fusion protein, the extracellular domain of OX40L may be any polypeptide including the extracellular domain of OX40L, for example, full-length OX40L. However, the claims should not be interpreted as being limited to these specific mechanisms.

**[Table 6]**

| SEQ ID NO: | Amino Acid Sequence |
|---|---|
| 24 | MTRLTVLALLAGLLASSRASSSPAKDPPIQRLRGAVTRCEDGQLFISSY KNEYQTMEVQNNSVVIKCDGLYIIYLKGSFFQEVKIDLHFREDHNPISIP MLNDGRRIVFTVVASLAFKDKVYLTVNAPDTLCEHLQINDGELIVVQLT PGYCAPEGSYHSTVNQVPLGGGGSGGGGSSERKALQTEMARIKKWL TFSGGGGSQDSEAAINRQINLELYASYVYLSMSYYFDRDDVALKNFAK YFLHQSHEEREHAEKLMKLQNQRGGRIFLQDIKKPDCDDWESGLNAM ECALHLEKNVNQSLLELHKLATDKNDPHLCDFIETHYLNEQVKAIKELG DHVTNLRKMGA |
| 25 | MTRLTVLALLAGLLASSRASGSQTCEDTLKTCSVIACGRDGRDGPKGE KGEPGQGLRGLQGPPGKLGPPGSVGSPGSPGPKGQKGDHGDNRAIE EKLANMEAEIRILKSKLQLTNKLHAFSMGGGGGSGGGGSSSSPAKDP PIQRLRGAVTRCEDGQLFISSYKNEYQTMEVQNNSVVIKCDGLYIIYLK GSFFQEVKIDLHFREDHNPISIPMLNDGRRIVFTVVASLAFKDKVYLTVN APDTLCEHLQINDGELIVVQLTPGYCAPEGSYHSTVNQVPL |

In Table 6, SEQ ID NOs: 24 and 25 are amino acid sequences obtained by translating the sequences of Materials 3 and 4, respectively.

## Claims

1. An mRNA molecule comprising a coding sequence for a polypeptide comprising an extracellular domain of OX40L, and a modified polyadenyl sequence, wherein the modified polyadenyl sequence comprises:
(a) a first sequence comprising one or more microRNA binding sequences;
(b) a second sequence located at 5' of the first sequence and comprising 20 or fewer consecutive A nucleotides'; and
(c) a third sequence located at 3' of the first sequence and comprising 80 or more consecutive A nucleotides.

2. The mRNA molecule of claim 1, wherein the polypeptide comprising the extracellular domain of OX40L is the extracellular domain of OX40L or a full-length OX40L.

3. The mRNA molecule of claim 1, wherein the polypeptide comprising the extracellular domain of OX40L is fused to one or more polypeptides that promote oligomerization of soluble OX40L trimers.

4. The mRNA molecule of claim 3, wherein the polypeptide comprising the extracellular domain of OX40L is fused to one or more polypeptides that promote oligomerization of soluble OX40L trimers via a linker.

5. The mRNA molecule of claim 3, wherein the one or more polypeptides that promote oligomerization are mannan-binding lectin (MBL), or ferritin, or a fragment thereof.

6. The mRNA molecule of claim 5, wherein the fragment of MBL is an alpha-helix region of the MBL.

7. The mRNA molecule of claim 1, wherein the microRNA binding sequence of the first sequence is tumor-specific.

8. The mRNA molecule of claim 7, wherein the microRNA is selected from the group consisting of miRNA-34a, miR-340, miR-133a, miR-34b, miR-19, miR-200, miR-15, miR-16, miR-1298, miR-663a, miR-449a, miR-138, miR-126, miR-10a, miR-10b, miR-140-3p, miR-140-5p, miR-204, miR-424, miR-193a-3p, miR-455-5p, miR-455-3p, miR-9, miR-10a, miR-148a, miR-488, miR-196a1, miR-182, miR-96, miR-193b, miR-27a, miR-196b, miR-10b, miR-29b2, miR-23a, miR-107, miR-542-3p, miR-93, miR-365a-4, miR-450a, miR-100, miR-105, miR-363, miR-105, miR-106b, miR-15b, miR-21, miR-376c, miR-93, miR-99b, miR-155, miR-33a, miR-876-3p, miR-362-3p, miR-25, let-7i, miR-423-3p, miR-16-2, miR-29a, miR-30d, miR-320, miR-181c, miR-128a, miR-21, let-7d, miR-450b-5p, miR-371-5p, miR-1245, miR-335, miR-492, miR-874, miR-30b, miR-193a-5p, miR-602, miR-346, miR-663, miR-25, miR-219-5p6, miR-184, miR-135a7, miR-584, miR-665, miR-638, miR-503, miR-628-3p, miR-381, miR-78, miR-92b, miR-149, miR-135b, miR-302d, miR-498, miR-766, miR-1389, miR-623, miR-519c-5p, miR-182, miR-494, miR-129-5p10, miR-513-5p, miR-200b, miR-634, miR-654-5p, miR-518b, miR-658, miR-373, miR-30c-2, miR-130a, miR-557, miR-551a, miR-637, miR-518c, miR-525-5p, miR-596, miR-552, miR-625, miR-183, miR-187, miR-544, miR-891a, miR-519e, miR-933, miR-939, miR-214, miR-671-5p, miR-137, miR-92b, miR-525-3p, miR-19a, and miR-409-5p.

9. The mRNA molecule of claim 1, comprising one or more modified nucleotides.

10. The mRNA molecule of claim 1, further comprising an expression control sequence.

11. The mRNA molecule of claim 10, wherein the expression control sequence comprises one or more selected from the group consisting of a Kozak sequence, a 5' UTR, and a 3' UTR.

12. The mRNA molecule of claim 11, wherein the 5' UTR is derived from an mRNA encoding a protein selected from the group consisting of p53, OX40L, albumin, serum amyloid A, apolipoprotein A/B/E, transferrin, alpha-fetoprotein, erythropoietin, factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen.

13. The mRNA molecule of claim 11, wherein the 3' UTR is derived from an mRNA encoding a protein selected from the group consisting of p53, OX40L, C3, CYP2E1, albumin, serum amyloid A, apolipoprotein A/B/E, transferrin, alpha-fetoprotein, erythropoietin, factor VIII, MyoD, myosin, myoglobin, myogenin, herculin, Tie-1, CD36, C/EBP, AML1, G-CSF, GM-CSF, CD11b, MSR, Fr-1, i-NOS, CD45, CD18, CD36, GLUT4, ACRP30, adiponectin, P-A/B/C/D, ORM1, HPX, FGA, CYP2E1, C3, ASC, APOA2, ALB, AGXT, α-globin, β-globin, tyrosine hydroxylase, and collagen.

14. The mRNA molecule of claim 1, wherein the modified polyadenyl sequence comprises 90 to 1125 nucleotides.

15. A composition for delivering an mRNA or a product expressed thereby to a subject, the mRNA comprising the mRNA according to any one of claims 1 to 14 as an active ingredient.

16. A method of delivering an mRNA or a product expressed thereby to a host cell, the method comprising introducing the mRNA according to any one of claims 1 to 14 into the host cell.

17. A nucleic acid molecule encoding the mRNA molecule according to any one of claims 1 to 14.

18. The nucleic acid molecule of claim 17, further comprising an expression control sequence operatively linked to the nucleic acid sequence, wherein the expression control sequence comprises a T7, T3, or SP6 promoter.

19. The nucleic acid molecule of claim 17, wherein the nucleic acid molecule is a nucleic acid construct or a vector.
